(19) Europäisches Patentamt
European Patent Office
Office européen des brevets

(11) Veröffentlichungsnummer: **0 552 657 A1**

(12) # EUROPÄISCHE PATENTANMELDUNG

(21) Anmeldenummer: **93100398.2**

(51) Int. Cl.$^5$: **A61K 31/215**

(22) Anmeldetag: **13.01.93**

(30) Priorität: **24.01.92 DE 4201903**

(43) Veröffentlichungstag der Anmeldung:
**28.07.93 Patentblatt 93/30**

(84) Benannte Vertragsstaaten:
**AT BE CH DE DK ES FR GB GR IE IT LI NL PT SE**

(71) Anmelder: **AMMON, Hermann P.T.**
**Im Kleeacker 30**
**W-7400 Tübingen(DE)**

(72) Erfinder: **Ammon, Hermann P. T., Prof. Dr. med.**
**Im Kleeacker 30**
**W-7400 Tübingen(DE)**
Erfinder: **Safayhi, Hasan, Dr. Chem.**
**Eichenweg 5**
**W-7400 Tübingen(DE)**
Erfinder: **Singh, G.B., Dr. Chem.**
**E.P. 441 Talab Khatika**
**Jammu-Tawi 180 001(IN)**

(74) Vertreter: **Kraus, Walter, Dr.**
**Patentanwälte Kraus, Weisert & Partner**
**Thomas-Wimmer-Ring 15**
**W-8000 München 22 (DE)**

(54) **Verwendung von reiner Boswelliasäure.**

(57) Die Erfindung betrifft die Verwendung reiner Boswelliasäure, eines physiologisch annehmbaren Salzes, eines Derivats, eines Salzes des Derivats oder einer Boswelliasäure enthaltenden pflanzlichen Zubereitung für die Prophylaxe und/oder die Bekämpfung von Entzündungsvorgängen, die durch gesteigerte Leukotrienbildung hervorgerufen werden, in der Human- oder Veterinärmedizin sowie die Verwendung reiner Boswelliasäure oder eines physiologisch annehmbaren Salzes, eines Derivats, eines Salzes des Derivats oder einer Boswelliasäure enthaltenden pflanzlichen Zubereitung zur Herstellung eines Arzneimittels für die Behandlung von Entzündungsvorgängen, die durch gesteigerte Leukotrienbildung hervorgerufen werden, in der Human- oder Veterinärmedizin.

EP 0 552 657 A1

Die Erfindung betrifft die Verwendung reiner Boswelliasäure, eines physiologisch annehmbaren Salzes, eines Derivats, eines Salzes des Derivats oder einer Boswelliasäure enthaltenden pflanzlichen Zubereitung für die Prophylaxe und/oder die Bekämpfung von Entzündungsvorgängen, die durch gesteigerte Leukotrienbildung hervorgerufen werden, in der Human- oder Veterinärmedizin.

Die Erfindung betrifft ebenfalls die Verwendung reiner Boswelliasäure oder eines physiologisch annehmbaren Salzes, eines Derivats, eines Salzes des Derivats oder einer Boswelliasäure enthaltenden pflanzlichen Zubereitung zur Herstellung eines Arzneimittels für die Behandlung von Entzündungsvorgängen, die durch gesteigerte Leukotrienbildung hervorgerufen werden, in der Human- oder Veterinärmedizin.

Erfindungsgemäß erfolgt die Verwendung insbesondere bei entzündlichen Gelenkerkrankungen, epidermalen Läsionen (Psoriasis), allergischem und chromischem Asthma, Endotoxinschock, entzündlichen Darmerkrankungen (Colitis ulcerosa, Morbus Crohn) und chronischer Hepatitis.

Entzündungsreaktionen sind Maßnahmen des Organismus, die dazu dienen, nach Schädigung eines Gewebes die den Schaden verursachenden Fremdkörper oder den geschädigten Teil des Gewebes zu beseitigen und durch Reparaturgewebe zu ersetzen. Eine Entzündung ist somit ein physiologischer Prozeß. Es gibt jedoch eine Reihe von Situationen, bei denen durch Entzündungsvorgänge zusätzlich Funktionen von Organen gestört werden und die dann therapeutischen Maßnahmen unterzogen werden müssen. Wichtige Entzündungen spielen sich ab zum Beispiel im Bereich von Gelenken (Rheumatismus), im Bereich der Bronchien und im Bereich des gesamten Magen-Darm-Kanals. Ein Weg der heutigen Therapie zur Beseitigung von Entzündungen ist die Verwendung von entzündungshemmenden Stoffen.

Entzündungen werden biochemisch ausgelöst durch die Freisetzung sogenannter Entzündungsmediatoren. Als solche spielen eine Rolle: Prostaglandine, Leukotriene, Histamin, Bradykinin und Komplement-Faktoren.

Die Synthese von Prostaglandinen und Leukotrienen erfolgt am Entzündungsort nach dem folgenden Schema:

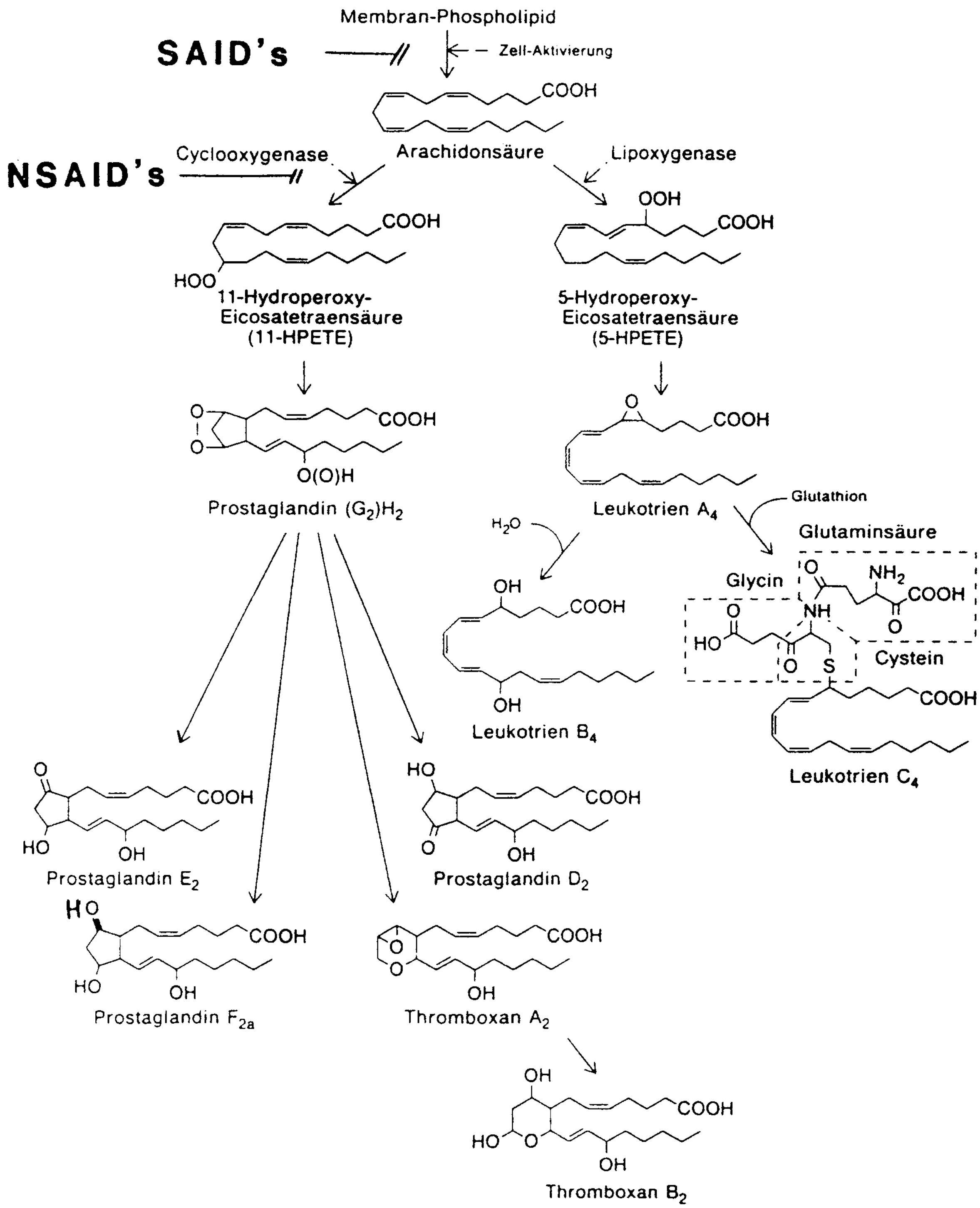


Aus diesem Schema geht hervor, daß es zwei wesentliche Arten von unterschiedlichen Entzündungsmediatoren gibt, die bei der Entstehung und Aufrechterhaltung von Entzündungen beteiligt sind: Prostaglandine und Leukotriene.

Prostaglandine haben im Entzündungsgeschehen folgende Funktionen: Veränderung des Gefäßtonus, Erhöhung der Gefäßpermeabilität, Schmerzauslösung, Regulation von Gerinnungsvorgängen und der Magensaftsekretion, Fieber. Leukotriene bewirken eine Kontraktion der Blutgefäße (Arterien) und der Bronchien, eine Erhöhung der Gefäßpermeabilität (Austritt von Ödemflüssigkeit) und die zielgerichtete Anlokkung von Leukozyten (vgl. Forth/Henschler/Rummel, Allgemeine und Spezielle Pharmakologie und Toxikologie, 5. Auflage, 1987, S. 176 bis 214 und 522 bis 546).

Die heutige Therapie der Entzündung geschieht mit Arzneimitteln, die vorwiegend in der Lage sind, die sogenannte Arachidonsäurekaskade, und zwar den Teil, der zur Bildung der Prostaglandine führt, zu

blockieren, und zwar durch Hemmung des Enzyms Cyclooxygenase. Die wichtigsten solcher Hemmstoffe sind: Acetylsalicylsäure und Derivate anderer schwacher Carbonsäuren, Pyrazolonderivate und Aminophenole.

Von diesen als Antiphlogistika (NSAID's = nichtsteroidale Antirheumatika) verwendeten Stoffen kann jedoch nur der linke Teil dieser Kaskade, nicht jedoch auch der Teil der Kaskade, der zur Bildung von Leukotrien führt, beeinflußt werden.

In dem genannten Schema ist die Stelle, wo diese Stoffe angreifen, mit "NSAID's" markiert. Diese nichtsteroidalen Antirheumatika haben somit bei entzündlichen Vorgängen, bei denen Leukotriene gebildet werden, keine oder fast keine Wirkung.

Eine andere Möglichkeit, Entzündungen zu behandeln, ist die Verwendung von Glucocorticoiden, insbesondere Cortison. Die Glucocorticoide werden als "SAID's" = steroidale Antirheumatika bezeichnet, und sie hemmen die Bildung der Phospholipase A2. Im obigen Schema ist die Stelle angegeben, wo die steroidalen Antirheumatika angreifen. Es ist ersichtlich, daß sie beide Teile der Arachidonsäurekaskade blockieren, d.h. einerseits die Prostaglandinbildung und andererseits die Leukotrienbildung hemmen. Bei vielen Krankheiten, bei denen eine Leukotrienbildung erfolgt, wie beispielsweise bei entzündlichen Gelenkerkrankungen, epidermalen Läsionen (Psoriasis) und allergischem und chronischem Asthma sowie entzündlichen Darmerkrankungen, ist jedoch eine längere Gabe von Corticoiden erforderlich. Ein großes Problem bei der Langzeitverwendung von Corticoiden stellen die allseits bekannten Nebenwirkungen auf Stoffwechsel und Hormonsysteme dar, die sich unter anderem auch in den Symptomen des Morbus Cushing äußern. Andere Nebenwirkungen sind das Vollmondgesicht, eine Stammfettsucht, Muskelschwäche, Hypertonie, Osteoporose, Magenbeschwerden und eine Behinderung der Immunvorgänge, um nur einige zu nennen. Die Verwendung von Glucocorticoiden ist auf Notfälle beschränkt. Glucocorticoide sind daher für eine Langzeitverwendung ungeeignet.

Bisher gibt es in der Therapie keinen einzigen Stoff, der in der Lage ist, das Entzündungsgeschehen selektiv auf der Seite der Leukotriensynthesehemmung zu beeinflussen. Das Vorliegen von solchen Stoffen wäre ein wesentlicher therapeutischer Fortschritt. In vielen Instituten der Welt wird derzeit nach derartigen Stoffen gesucht. Es wurde auch eine Reihe solcher Stoffe gefunden, doch hat sich bei allen herausgestellt, daß sie zu toxisch sind, um für die Therapie in Frage zu kommen.

In der Ayurvedischen Medizin Indiens werden Arzneimittel, die Präparationen aus der Pflanze Boswellia serrata enthalten, zur Behandlung von Entzündungen, aber auch Rheumatismus verwendet. Es war bisher noch nicht bekannt, vor allen Dingen aber nicht wissenschaftlich nachgewiesen, welcher der Inhaltsstoffe dieser Arzneipflanze den antiphlogistischen Effekt ausübt. Insbesondere war nicht bekannt, wie die verwendeten Extrakte wirken, und diese Arzneimittel wurden vorwiegend bei den Krankheiten eingesetzt, bei denen Prostaglandine als Entzündungsmediatoren gebildet werden. Die Verwendung dieser Arzneimittel bei Krankheiten, bei denen Leukotriene gebildet werden, ist in der Literatur nicht beschrieben.

Bei Morbus Crohn und bei der Colitis ulcerosa handelt es sich um zwei chronisch entzündliche Darmerkrankungen, die mit der bisher zur Verfügung stehenden Therapie nicht oder nur ungenügend beeinflußt werden können. Pathophysiologisch gesehen wird angenommen, daß eine übermäßige Bildung sogenannter Leukotriene (Entzündungsmediatoren, die insbesondere als Lockstoffe für weiße Blutzellen dienen) die Hauptursache dafür sind, daß diese Erkrankungen am Laufen gehalten werden. Bisher gibt es praktisch kein wirksames Medikament gegen entzündliche Darmkrankheiten. Zur Verfügung stehen zwar sogenannte Glucocorticoide (Derivate von Nebennierenrindenhormonen), insbesondere Cortison. Sie sind jedoch, wie oben ausgeführt, mit erheblichen Nebenwirkungen behaftet, so daß ihr Einsatz nicht unproblematisch ist. Auch die Anwendung von Sulfasalazin ist unbefriedigend. Sulfasalazin ist ein Stoff, der hauptsächlich die Bildung sogenannter Prostaglandine (ebenfalls Mediatoren der Entzündung) hemmt. Durch Hemmung der Prostaglandinsynthese kommt man bei diesen Erkrankungen nicht immer zum Ziel.

Chronisches Asthma und Psoriasis sind Krankheiten, für die bis heute keine befriedigend wirksamen Arzneimittel zur Verfügung stehen. Beide Krankheiten werden u.a. mit Cortison oder Cortisonderivaten behandelt. Die Verabreichung von Cortison und seinen Derivaten, insbesondere während längerer Zeit, ist jedoch mit erheblichen Nachteilen verbunden. Auch chronische Hepatitis kann nicht zufriedenstellend behandelt werden.

Der vorliegenden Erfindung liegt die Aufgabe zugrunde, die Verwendung von Präparaten zur Verfügung zu stellen, die zur Prophylaxe und/oder Bekämpfung von Krankheiten, die mit einer übermäßigen Bildung von Leukotrienen einhergehen, dienen. Das erfindungsgemäß zur Verfügung gestellte Präparat soll in der Lage sein, das Entzündungsgeschehen selektiv auf der Seite der Leukotriensynthesehemmung zu beeinflussen. Erfindungsgemäß soll insbesondere ein Weg aufgezeigt werden, um die steroidalen Antirheumatika ersetzen zu können, und es sollen Möglichkeiten zur Verfügung gestellt werden, gemäß denen ein Arzneimittel, welches die Leukotriensynthese hemmt, während langer Zeit verabreicht werden kann, ohne

daß irgendwelche Nebenwirkungen und insbesondere die Nebenwirkungen der steroidalen Antirheumatika auftreten. Erfindungsgemäß soll die Verwendung eines Arzneimittels zur Verfügung gestellt werden, welches bei chronischen Entzündungskrankheiten eingesetzt werden kann und welches insbesondere bei entzündlichen Gelenkerkrankungen, epidermalen Läsionen (Psoriasis), allergischem und chronischem Asthma, Endotoxinschock, entzündlichen Darmerkrankungen (Colitis ulcerosa, Morbus Crohn) und chronischer Hepatitis wirkt. Das erfindungsgemäß zur Verfügung gestellte Arzneimittel soll untoxisch sein und von den Patienten gut toleriert werden. Gerade zur Behandlung der genannten Krankheiten wird von der pharmazeutischen Industrie fieberhaft nach Leukotriensynthesehemmern gesucht, die untoxisch sind.

Überraschenderweise wurde jetzt gefunden, daß Boswelliasäure, ein physiologisch annehmbares Salz, ein Derivat, ein Salz des Derivats oder eine Boswelliasäure enthaltende pflanzlische Zubereitung für die Prophylaxe und/oder die Bekämpfung von Entzündungsvorgängen, die durch gesteigerte Leukotrienbildung hervorgerufen werden, in der Human- oder Veterinärmedizin wirkt.

Mit Hilfe des Testes mit Calcium- und Ionophor-stimulierten neutrophilen Leukozyten der Ratte konnte nachgewiesen werden, daß Extrakte aus Boswellia serrata vorwiegend die Bildung von Leukotrienen blockieren, dagegen die Prostaglandinsynthese erst in wesentlich höheren Konzentrationen beeinflussen (vgl. zur Methodik: a) Messung der Leukotriene: Safayhi et al., Biochem. Pharmacology, 34:2691-2694, 1985; b) Messung der Prostaglandine: Kommerzielle Radio-Immunoassay-Kits der Firma Amersham für 6-Keto-PGF1$\alpha$).

Die Pflanze Boswellia serrata muß daher über Inhaltsstoffe verfügen, die gerade in der Lage sind, die Arachidonsäurekaskade auf der Seite der Leukotrienbildung einigermaßen selektiv zu inhibieren. Boswellia serrata enthält nach Angaben von Pardhy & Bhattacharyya (Ind. J. Chem., 16B:176-178, 1978) im wesentlichen folgende Inhaltsstoffe: $\beta$-Boswelliasäure, Acetyl-$\beta$-boswelliasäure, Acetyl-11-keto-$\beta$-boswelliasäure, 11-Keto-$\beta$-boswelliasäure, über die bisher jedoch keine pharmakologischen Untersuchungen im Bereich einer Entündungsverhinderung durchgeführt wurden. Aufgrund der Literaturstelle G.B. Singh und C.K. Atal, Agents and Actions, Bd. 18, S. 410, hätte man erwarten müssen, daß Boswelliasäure keinen Einfluß auf Entzündungen hat, welche durch Leukotriene hervorgerufen werden. In dieser Literaturstelle wird auf Seite 410, rechte Spalte, ausgeführt, daß Salai guggal, ein Extrakt, der aus Boswellia serrata gewonnen wird, keine Wirkung bei dem Baumwollpellet-induzierten Granulomatest aufweist, der für die oben erwähnten SAID's Arzneimittel charakteristisch ist.

Die Strukturformeln von Boswelliasäure und einigen ihrer Derivate werden im folgenden aufgeführt:

(A):

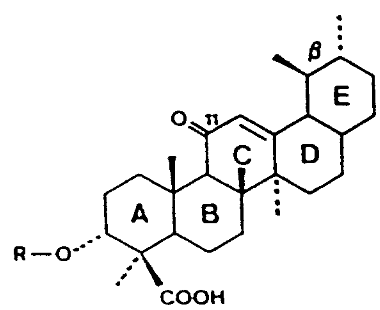

R = H : 11-Keto-$\beta$-boswelliasäure
R = Acetyl : Acetyl-11-keto-$\beta$-boswelliasäure
R = Formyl : Formyl-11-keto-$\beta$-boswelliasäure

(B):

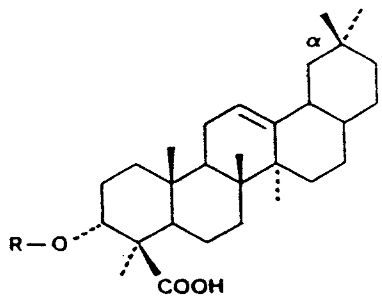

R = H : α-Boswelliasäure
R = Acetyl : Acetyl-α-boswelliasäure
R = Formyl : Formyl-α-boswelliasäure

Als Boswelliasäure wird vorzugsweise β-Boswelliasäure verwendet, die nach Literaturangaben aus Boswellia serrata oder anderen bekannten Boswelliasäure enthaltenden Pflanzen isoliert wird. Die β-Boswelliasäure kann geringe Mengen an α- oder -Boswelliasäure enthalten. Als physiologisch annehmbare Salze der Boswelliasäure können die Natrium-, Kalium-, Ammonium-, Calciumsalze verwendet werden. Als Derivate der Boswelliasäure können niedere Alkylester, die durch Veresterung der Carboxylgruppe mit einem $C_1$-$C_6$-Alkohol erhalten werden, vorzugsweise der Methylester, oder Ester, die durch Veresterung der Hydroxylgruppe mit einer physiologisch verträglichen Carbonsäure erhalten werden, verwendet werden. Bevorzugte Derivate sind β-Boswelliasäureacetat, β-Boswelliasäureformiat, β-Boswelliasäuremethylester, Acetyl-β-boswelliasäure, Acetyl-11-keto-β-boswelliasäure und 11-Keto-β-boswelliasäure.

Erfindungsgemäß ist es weiterhin möglich, eine Boswelliasäure enthaltende pflanzliche Zubereitung zu verwenden. Erfindungsgemäß werden Präparate, die aus dem Harz gewonnen werden, verwendet.

Pflanzen, die Boswelliasäure (syn.: Boswellinsäure) enthalten, sind:
Boswellia (serrata, papyrifera, frereana, carteri, thurifera, glabra, bhaw-dajiana, oblongata, socotrana und andere Vertreter dieser Familie), vornehmlich das Harz.

Erfindungsgemäß ist es weiterhin möglich, daß die Verwendung zusammen mit anderen chemisch reinen Arzneistoffen und/oder anderen pflanzlichen Arzneimitteln erfolgt. Beispiele für solche andere chemisch reine Arzneistoffe sind:

BRONCHOLYTIKA UND ANTIASTHMATIKA

SYMPATHOMIMETIKA:

- Carbuterol-HCl
- Clenbuterol-HCl
- Fenoterol-HBr
- Isoetarin-HCl
- Orciprenalinsulfat
- Pirbuterol-HCl
- Procaterol-HCl
- Reproterol-HCl
- Sabutamolsulfat
- Terbutalinsulfat
- Tulobuterol-HCl

ANTIPSORIATIKA

NICHTSTEROIDALE ANTIPHLOGISTIKA:

- Salicylsäure und Derivate

VITAMINE:

- Folsäure
- Vitamin E

- Vitamin B12
- Vitamin A

VERSCHIEDENE:

- Cadmiumsulfid
- Benzalkoniumchlorid
- Natriumbituminosulfonat
- Ammoidin
- Allantoin
- Methotrexat
- Paraffin
- Tioxolon
- Dithranol
- Fumarsäure
- Undecylensäure
- Polyoxyethylenlaurylethersulfat
- Etretinat
- Zinkoxid
- Harnstoff
- Milchsäure

NICHSTEROIDALE ANTIRHEUMATIKA

PYRAZOL-DERIVATE:

- Azapropazon
- Bumadizon
- Famprofazon
- Mofebutazon
- Nifenazon
- Oxyphenbutazon
- Phenylbutazon
- Pyrazinobutazon

ARYLESSIGSÄURE-DERIVATE UND INDOL-DERIVATE:

- Acemetacin
- Bufexamac
- Diclofenac
- Indometacin
- Lonazolac
- Proglumetacin
- Tolmetin

ANTHRANILSÄURE-DERIVATE:

- Flufenaminsäure
- Mefenaminsäure
- Nifluminsäure

ARYLPROPIONSÄURE-DERIVATE:

- Carprofen
- Fenoprofen
- Fenbufen
- Flurbiprofen
- Ibuprofen

- Ketoprofen
- Naproxen
- Piroxicam
- Pirprofen
- Tiaprofensäure

OXICAME:

- Tenoxicam

SONSTIGE:

- Benzydamin
- Benfotiamin
- Chloroquin
- Hydroxychloroquin
- Auranofin
- (1-D-Glukosylthio)gold
- Aurothiomalat
- Aurothiopolypeptid
  (= Goldkeratinat)
- Tetrachlorogold(III)-säure
- D-Penicillamin
- Hyaluronidase
- Nabumeton
- Etofenamat
- Ademetionin
- Serrapeptase
- Azathioprin
- Chlorambucil
- Cyclophosphamid
- Methotrexat
- Glucosaminsulfat
- Penicillin
- Bienengiftpräparat
- Schwefel
- Oxaceprol
- Orgotein
- Sulfasalazin
  (= Salazosulfapyridin)

Die Boswellisäuren hemmen die Aktivität der 5-Lipoxygenase und damit die Entstehung der Entzündungsmediatoren vom Leukotrien-Typ. Sie hemmen die 5-Lipoxygenase spezifisch, d.h. weder die 12-Lipoxygenase (12-HETE-Bildung) noch die Cyclooxygenase-Aktivität (Prostanoid-Bildung) werden durch die Boswelliasäuren beeinträchtigt. Im Gegensatz zu vielen experimentell eingesetzten 5-Lipoxygenase-Inhibitoren (zum Beispiel NDGA, BW755c etc.), die aufgrund ihrer reduzierenden Wirkung potentiell alle oxidierenden Enzyme nichtselektiv hemmen könnten, besitzen die Boswelliasäuren keine reduzierenden Eigenschaften. Ein solcher spezifischer Hemmstoff der 5-Lipoxygenase, der aufgrund seiner hohen Selektivität auch für eine in vivo parenterale Verabreichung bei Leukotrien-vermittelten Krankheiten geeignet erscheint, ist auf dem Markt nicht verfügbar.

Den Angriffsort/Wirkmechanismus der Boswelliasäuren verdeutlich die folgende Abbildung:

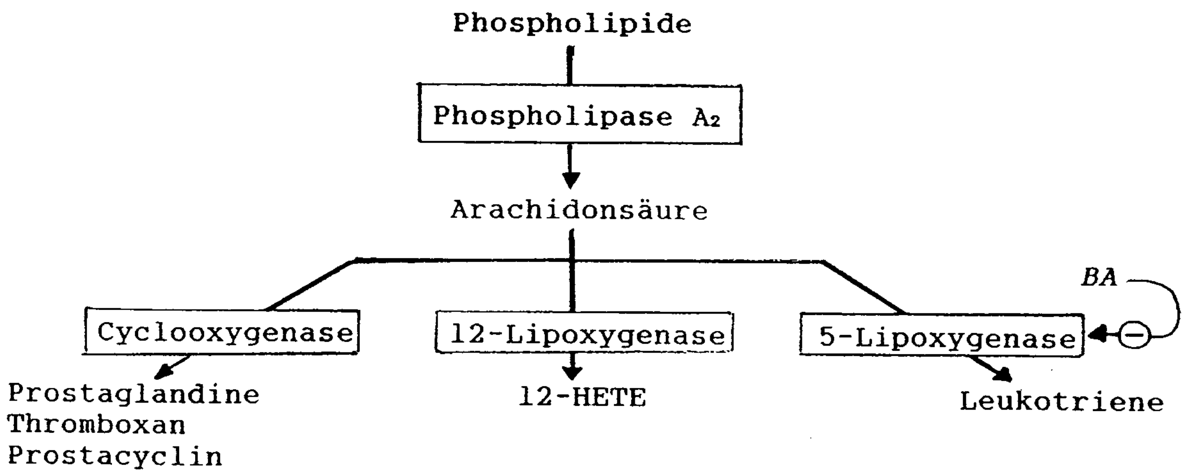


<u>TABELLE</u>: Hemmung der 5-Lipoxygenase durch Boswelliasäuren

Folgende Boswelliasäuren (syn.: Boswellinsäuren) bzw. Derivate wurden isoliert und zeigten eine 5-Lipoxygenase-hemmende Wirkung:

| Boswelliasäuren | $IC_{50}$-Werte für die 5-LO-Hemmung (µM) |
|---|---|
| ß-Boswelliasäure | 5 |
| Acetyl-boswelliasäure | 7 |
| 11-Keto-ß-boswelliasäure | 5 |
| Acetyl-11-keto-ß-boswelliasäure | 2 |
| α-Boswelliasäure | 5 |
| Acetyl-α-boswelliasäure | 7 |

Anhand der beigefügten Zeichnungen wird die Erfindung näher erläutert. Es zeigen:

Fig. 1 die 5-Lipoxygenase-Aktivität in isolierten, stimulierten neutrophilen Granulocyten aus dem Peritoneum der Ratte in Gegenwart steigender Konzentrationen von Acetyl-11-keto-β-boswelliasäure (●), Nordihydroguaiaretsäure (■) und Hydrocortison (▲). Methode: Safayhi, Tiegs, Wendel 1985.

Fig. 2 die 12-Lipoxygenase-Aktivität in isolierten, stimulierten Thrombocyten des Menschen in Gegenwart steigender Konzentrationen von Acetyl-boswelliasäure (●), Nordihydroguaiaretsäure (■) und Indometacin (□). Methode: Safayhi et al., JPET accepted.

Fig. 3 die Cyclooxygenase-Aktivität in isolierten, stimulierten Thrombocyten des Menschen in Gegenwart steigender Konzentrationen von Acetyl-boswelliasäure (●), Nordihydroguaiaretsäure (■) und Indometacin (□). Methode: Safayhi et al., JPET accepted.

Fig. 4 die Elutionsprofile der durch Eisen/Ascorbat-Stimulation aus exogener Arachidonsäure im zellfreien System entstehenden oxidierten und hydroxylierten Produkte in Gegenwart steigender Konzentrationen von Acetyl-boswelliasäure (A) und Nordihydroguaiaretsäure (B). Methode: Safayhi et al., JPET accepted.

Erfindungsgemäß können alle Krankheiten, welche mit Leukotrienbildung einhergehen, behandelt werden. Es ist jedoch bevorzugt, als Entzündungen entzündliche Gelenkerkrankungen, epidermale Läsionen (Psoriasis), allergisches und chronisches Asthma, den Endotoxinschock und entzündliche Darmerkrankungen, wie Colitis ulcerosa und Morbus Crohn, und chronische Hepatitis, zu behandeln.

Erfindungsgemäß wird die Boswelliasäure je nach Bedarf verabreicht. Da sie wenig toxisch ist, ist die Dosierung nicht kritisch und kann in Abhängigkeit von der Schwere der Krankheit, dem Gewicht des zu behandelnden Patienten und der Dauer der Behandlung vom Arzt leicht variiert werden.

Einheitsdosen können beispielsweise ein- bis viermal täglich verabreicht werden. Die exakte Dosis hängt vom Verabreichungsweg und dem zu behandelnden Zustand ab. Naturgemäß kann es erforderlich sein, Routinevariationen der Dosis, je nach dem Alter und dem Gewicht des Patienten sowie der Schwere des zu behandelnden Krankheitszustandes, vorzunehmen.

Die erfindungsgemäß verwendeten Zubereitungen können in an sich bekannter Weise unter Verwendung eines oder mehrerer pharmazeutisch annehmbarer Träger oder Verdünnungsmittel formuliert werden. Die Zubereitungen können für die orale, parenterale, rektale oder intranasale Verabreichung oder in einer für die Verabreichung durch Inhalation oder Insufflation geeigneten Weise formuliert werden. Zubereitungen der Verbindungen für die orale Verabreichung sind bevorzugt.

Die pharmazeutischen Zubereitungen für die orale Verabreichung können in Form von beispielsweise Tabletten oder Kapseln, die nach an sich bekannten Verfahren mit pharmazeutisch annehmbaren Verdünnungsmitteln, wie Bindemitteln (zum Beispiel vorgelatinierter Maisstärke, Polyvinylpyrrolidon oder Hydroxypropylmethylcellulose), Füllstoffen (zum Beispiel Lactose, Saccharose, Mannit, Maisstärke, mikrokristalline Cellulose oder Calciumhydrogenphosphat); Schmiermitteln (zum Beispiel Stearinsäure, Polyethylenglykol, Magnesiumstearat, Talk oder Siliciumdioxid); Desintegrationsmitteln (zum Beispiel Kartoffelstärke, Natriumstärkeglykolat oder Natriumcarboxymethylcellulose); oder Benetzungsmitteln (zum Beispiel Natriumlaurylsulfat), hergestellt werden, vorliegen. Die Tabletten können nach an sich bekannten Verfahren überzogen werden. Flüssige Zubereitungen für die orale Verabreichung können in Form von beispielsweise wäßrigen oder öligen Lösungen, Sirupen, Elixieren, Emulsionen oder Suspensionen vorliegen, oder sie können als Trockenprodukt für die Konstitution mit Wasser oder einem anderen geeigneten Träger vor der Verwendung vorliegen. Solche flüssigen Zubereitungen können nach an sich bekannten Verfahren mit pharmazeutisch annehmbaren Zusatzstoffen hergestellt werden, wie Suspensionsmitteln (zum Beispiel Sorbitsirup, Cellulosederivaten, Glucose/Zucker-Sirup, Gelatine, Aluminiumstearatgel oder hydrierten genießbaren Fetten); Emulgiermitteln (zum Beispiel Lecithin, Gummi arabicum oder Sorbitan-monooleat); nichtwäßrigen Trägern (zum Beispiel Mandelöl, öligen Estern, Ethylalkohol oder fraktionierten Pflanzenölen); und Konservierungsmitteln (zum Beispiel Methyl- oder Propyl-p-hydroxybenzoaten oder Sorbinsäure). Die flüssigen Zubereitungen können auch an sich bekannte Puffer, Geschmacks- bzw. Aromamittel, Farbstoffe und Süßstoffe, je nach Bedarf, enthalten.

Für die parenterale Verabreichung können die Verbindungen für Injektion, bevorzugt intravenöse, intramuskuläre oder subkutane Injektion, formuliert werden. Zubereitungen für die Injektion können in Eindosenform, zum Beispiel in Ampullen, oder in Mehrfachdosis-Behältern mit einem zugegebenen Konservierungsstoff vorliegen. Die Zubereitungen können in Form von Suspensionen, Lösungen oder Emulsionen in öligen oder wäßrigen Trägern vorliegen und Zubereitungshilfsmittel enthalten, wie Suspendier-, Stabilisier- und/oder Dispersionsmittel, und/oder Mittel zur Einstellung der Tonizität der Lösung enthalten. Alternativ kann der aktive Bestandteil in Pulverform für die Konstitution mit einem geeigneten Träger, zum Beispiel sterilem pyrogenfreien Wasser, vor der Verwendung vorliegen.

Die Verbindungen können ebenfalls als rektale Zubereitungen, wie Suppositorien, formuliert werden, zum Beispiel solche, die an sich bekannte Suppositorien-Grundstoffe, wie Kakaobutter oder andere Glyceride, enthalten.

Für die intranasale Verabreichung können die Verbindungen als flüssige Sprays, in Form von Tropfen oder als Schnupfpulver verwendet werden.

Für die Verabreichung durch Inhalation werden die Verbindungen zweckdienlich in Form eines Aerosolsprays aus einer unter Druck stehenden Packung unter Verwendung geeigneter Treibmittel oder in einer Zerstäubungsvorrichtung abgegeben. Im Falle eines unter Druck stehenden Aerosols wird die Dosiseinheit bestimmt, indem ein Ventil vorgesehen wird, welches eine abgemessene Menge freigibt. Kapseln und Patronen aus beispielsweise Gelatine für die Verwendung in einer Inhaltionsvorrichtung oder einer Insufflationsvorrichtung können so zubereitet werden, daß sie ein Pulvergemisch aus einer erfindungsgemäß verwendeten Verbindung und einem geeigneten Pulver-Grundstoff, wie Lactose oder Stärke, enthalten.

Die folgenden Beispiele erläutern die erfindungsgemäße Verwendung.

**Beispiel 1**

Tabletten für die orale Verabreichung

A. Direkte Kompression

(1)

| | |
|---|---|
| Wirkstoff: Boswelliasäure | 15 - 30 mg/Tablette |
| (bzw. pulverisierte Droge | 0,5 - 1,0 g/Tablette) |
| Magnesiumstearat BP | 0,65 mg/Tablette |
| wasserfreie Lactose | 80 mg/Tablette |

Der Wirkstoff wird mit der wasserfreien Lactose und dem Magnesiumstearat vermischt, und das Gemisch wird gesiebt. Das entstehende Gemisch wird zu Tabletten unter Verwendung einer Tablettiermaschine verpreßt.

(2)

| | |
|---|---|
| Wirkstoff: Boswelliasäure | 15 - 30 mg/Tablette |
| (bzw. pulverisierte Droge | 0,5 - 1,0 g/Tablette) |
| Magnesiumstearat BP | 0,7 mg/Tablette |
| mikrokristalline Cellulose NF | 100 mg/Tablette |

Der Wirkstoff wird gesiebt und mit der mikrokristallinen Cellulose und dem Magnesiumstearat vermischt. Das entstehende Gemisch wird unter Verwendung einer Tablettiermaschine zu Tabletten verpreßt.

B. Nasse Granulierung

| | |
|---|---|
| Wirkstoff: Boswelliasäure | 15 - 30 mg/Tablette |
| (bzw. pulverisierte Droge | 0,5 - 1,0 g/Tablette) |
| Lactose BP | 150,0 mg/Tablette |
| Stärke BP | 30,0 mg/Tablette |
| vorgelatinierte Maisstärke BP | 15,0 mg/Tablette |
| Magnesiumstearat BP | 1,5 mg/Tablette |

Der Wirkstoff wird durch ein geeignetes Sieb gesiebt und mit der Lactose, der Stärke und der vorgelatinierten Maisstärke vermischt. Geeignete Volumina an gereinigtem Wasser werden zugegeben, und das Pulver wird granuliert. Nach dem Trocknen wird das Granulat gesiebt und mit dem Magnesiumstearat vermischt. Das Granulat wird dann unter Verwendung von Lochstanzen mit geeignetem Durchmesser zu Tabletten verpreßt.

Tabletten anderer Zusammensetzung können hergestellt werden, indem man das Verhältnis von Wirkstoff zu Lactose oder das Kompressionsgewicht ändert und entsprechende Lochstanzen verwendet.

**Beispiel 2**

| Kapseln | |
|---|---|
| Wirkstoff: Boswelliasäure | 15 - 30 mg/Kapsel |
| (bzw. granulierte Droge | 0,5 - 1,0 g/Kapsel) |
| freifließende Stärke | 150,00 mg/Kapsel |
| Magnesiumstearat BP | 1,00 mg/Kapsel |

Der Wirkstoff wird gesiebt und mit den anderen Bestandteilen vermischt. Das Gemisch wird unter Verwendung einer geeigneten Vorrichtung in Hartgelatinekapseln Nr. 2 gefüllt. Andere Kapseln können hergestellt werden, indem man das Füllgewicht ändert und erforderlichenfalls die Kapselgröße entsprechend ändert.

**Beispiel 3**

Sirup

| Saccharose-freie Zubereitung | | mg/5 ml Dosis |
|---|---|---|
| Wirkstoff: Boswelliasäure | | 15 - 30 |
| Hydroxypropylmethylcellulose USP (Viskositäts-Typ 4000) | | 22,5 |
| Puffer ) | | |
| Geschmacksstoff ) | | |
| Farbstoff ) | | nach Bedarf |
| Konservierungsmittel ) | | |
| Süßstoff ) | | |
| gereinigtes Wasser | auf | 5,0 ml |

Die Hydroxypropylmethylcellulose wird in heißem Wasser dispergiert, abgekühlt und dann mit einer wäßrigen Suspension vermischt, die den Wirkstoff und die anderen Komponenten der Zubereitung enthält. Die entstehende Lösung wird auf ihr Volumen eingestellt und vermischt.

**Beispiel 4**

| Suspension | | mg/5 ml Dosis |
|---|---|---|
| Wirkstoff: Boswelliasäure | | 15 - 30 |
| (bzw. pulverisierte Droge | | 0,5 - 1,0 g) |
| (getrockneter Drogenextrakt entsprechend) | | |
| Aluminiummonostearat | | 75,00 |
| Süßstoff ) | | |
| Geschmacksstoff ) | | nach Bedarf |
| Farbstoff ) | | |
| fraktioniertes Kokosnußöl | auf | 5,00 |

Das Aluminiummonostearat wird in etwa 90% des fraktionierten Kokosnußöls dispergiert. Die resultierende Suspension wird unter Rühren auf 115 °C erhitzt und dann abgekühlt. Die Süß-, Geschmacks- und Farbstoffe werden zugesetzt, und der Wirkstoff wird dispergiert. Die Suspension wird mit dem restlichen fraktionierten Kokosnußöl auf das Volumen eingestellt und vermischt.

**Beispiel 5**

| Sublinguale Tablette | |
|---|---|
| Wirkstoff: Boswelliasäure<br>(bzw. Drogenextrakt<br>verpreßbarer Zucker NF<br>Magnesiumstearat BP | 15 - 30 mg/Tablette<br>0,5 - 1,0 g/Tablette)<br>50,5 mg/Tablette<br>0,5 mg/Tablette |

Der Wirkstoff wird durch ein geeignetes Sieb gesiebt, mit den anderen Bestandteilen vermischt und unter Verwendung geeigneter Lochstanzen verpreßt. Tabletten anderer Stärke können hergestellt werden, indem man das Verhältnis von Wirkstoff zu Träger oder das Kompressionsgewicht ändert.

**Beispiel 6**

| Suppositorien für die rektale Verabreichung | |
|---|---|
| Wirkstoff: Boswelliasäure<br>Witepsol H15+ auf | 15 - 30 mg<br>1,0 g |

+ geeignete Qualität von Adeps solidus Ph.Eur.

Eine Suspension des Wirkstoffs in geschmolzenem Witepsol wird hergestellt und unter Verwendung einer geeigneten Vorrichtung in 1-g-Suppositorienformen eingefüllt.

**Beispiel 7**

| Injektion für intravenöse Verabreichung | |
|---|---|
| Wirkstoff: Boswelliasäure | 15 - 30 mg/ml |
| Natriumchlorid-intravenöse Infusion, BP, 0,9% Gew./Vol. auf | 1 ml |
| Ansatzgröße 2500 ml | |

Der Wirkstoff wird in einem Teil der Natriumchlorid-intravenösen Infusion gelöst, die Lösung mit der Natriumchloridintravenösen Infusion auf das Volumen eingestellt und die Lösung gründlich vermischt. Die Lösung wird in klare, Typ 1, 10-ml-Glasampullen eingefüllt und unter Stickstoff im Kopfraum durch Abschmelzen des Glases abgesiegelt. Die Ampullen werden durch Erhitzen im Autoklaven bei 120 °C für nicht kürzer als 20 Minuten sterilisiert.

**Beispiel 8**

| Patrone für die Inhalation | |
|---|---|
| Wirkstoff (mikronisiert): Boswelliasäure<br>Lactose BP | 15 - 30 mg/Patrone<br>25,00 |

Der Wirkstoff wird in einer Strahlmühle zu einem feinen Teilchengrößenbereich mikronisiert und dann mit der Lactose vermischt. Die Pulvermischung wird in Hartgelatinekapseln Nr. 3 eingefüllt.

**Beispiel 9**

Nasenspray

| | | | |
|---|---|---|---|
| Wirkstoff: Boswelliasäure | | | 1,5 - 3,0 %/Vol. |
| Konservierungsmittel | ) | | nach Bedarf |
| Natriumchlorid BP | ) | | |
| gereinigtes Wasser BP | | auf | 100 |
| Abgabegewicht | | | 100 mg (Äquivalent zu 7 mg Wirkstoff) |

Der Wirkstoff, der Konservierungsstoff und das Natriumchlorid werden in einem Teil des Wassers gelöst. Die Lösung wird mit Wasser auf das Volumen eingestellt und die Lösung gründlich vermischt.

**Patentansprüche**

1. Verwendung von reiner Boswelliasäure, eines physiologisch annehmbaren Salzes, eines Derivats, eines Salzes des Derivats oder einer Boswelliasäure enthaltenden pflanzlichen Zubereitung für die Prophylaxe und/oder die Bekämpfung von Entzündungsvorgängen, die durch gesteigerte Leukotrienbildung hervorgerufen werden, in der Human- oder Veterinärmedizin.

2. Verwendung nach Anspruch 1, dadurch **gekennzeichnet,** daß die Verwendung bei entzündlichen Gelenkerkrankungen, epidermalen Läsionen (Psoriasis), allergischem und chromischem Asthma, Endotoxinschock, entzündlichen Darmerkrankungen (Colitis ulcerosa, Morbus Crohn) und chronischer Hepatitis erfolgt.

3. Verwendung nach einem der Ansprüche 1 oder 2, dadurch **gekennzeichnet,** daß die Verwendung intraperitoneal, oral, bukkal, rektal, intramuskulär, topisch, subkutan, intraartikulär oder intravenös erfolgt.

4. Verwendung nach einem der Ansprüche 1, 2 oder 3, dadurch **gekennzeichnet,** daß die Verwendung in Form von Tabletten, Dragees, Kapseln, Lösungen, Emulsionen, Salben, Cremes, Inhalationspräparaten, Aerosolen oder Suppositorien erfolgt.

5. Verwendung reiner Boswelliasäure oder eines physiologisch annehmbaren Salzes, eines Derivats, eines Salzes des Derivats oder einer Boswelliasäure enthaltenden pflanzlichen Zubereitung zur Herstellung eines Arzneimittels für die Behandlung von Entzündungsvorgängen, die durch gesteigerte Leukotrienbildung hervorgerufen werden, in der Human- oder Veterinärmedizin.

6. Verwendung nach Anspruch 5, dadurch **gekennzeichnet,** daß ein Arzneimittel für die Behandlung von entzündlichen Gelenkerkrankungen, epidermalen Läsionen (Psoriasis), allergischem oder chronischem Asthma, Endotoxinschock, entzündlichen Darmerkrankungen (Colitis ulcerosa, Morbus Crohn) und chronischer Hepatitis hergestellt wird.

7. Verwendung nach einem der Ansprüche 5 oder 6, dadurch **gekennzeichnet,** daß die Verwendung zur Herstellung eines Arzneimittels für die intraperitoneale, orale, bukkale, rektale, intrasmukuläre, topische, subkutane, intraartikuläre oder intravenöse Verabreichung erfolgt.

8. Verwendung nach einem der Ansprüche 5, 6 oder 7, dadurch **gekennzeichnet,** daß die Verwendung zur Herstellung eines Arzneimittels in Form von Tabletten, Dragees, Kapseln, Lösungen, Emulsionen, Salben, Cremes, Inhalationspräparaten, Aerosolen oder Suppositorien erfolgt.

9. Verwendung nach mindestens einem der Ansprüche 1 bis 8, dadurch **gekennzeichnet,** daß die Verwendung zusammen mit anderen chemisch reinen Arzneistoffen und/oder pflanzlichen Arzneimitteln erfolgt.

FIG. 1

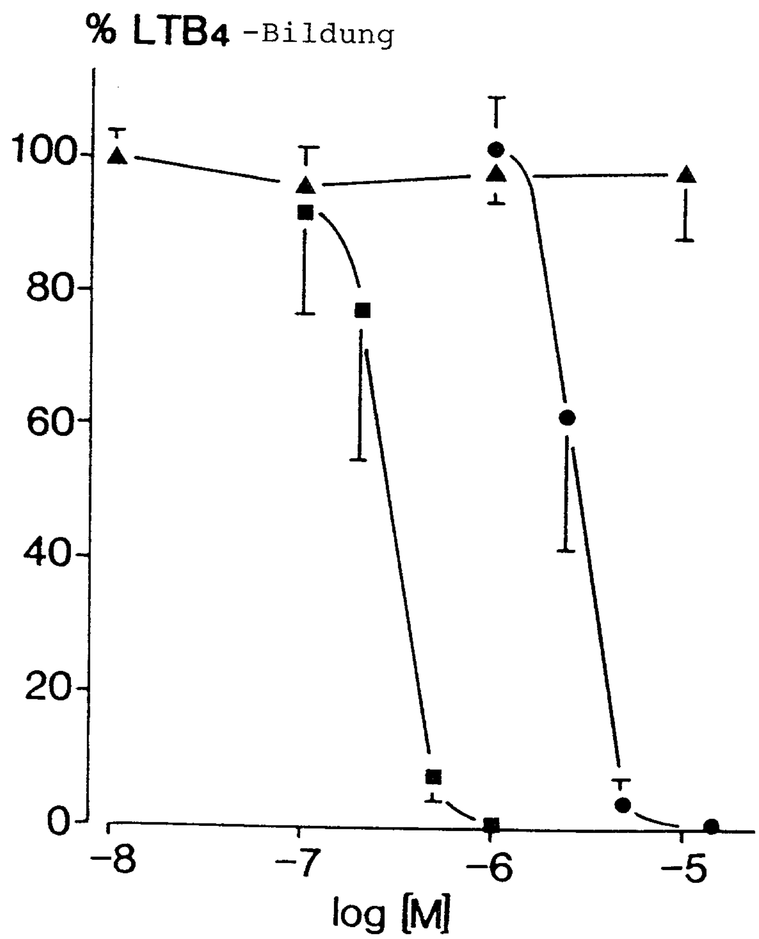

FIG. 2

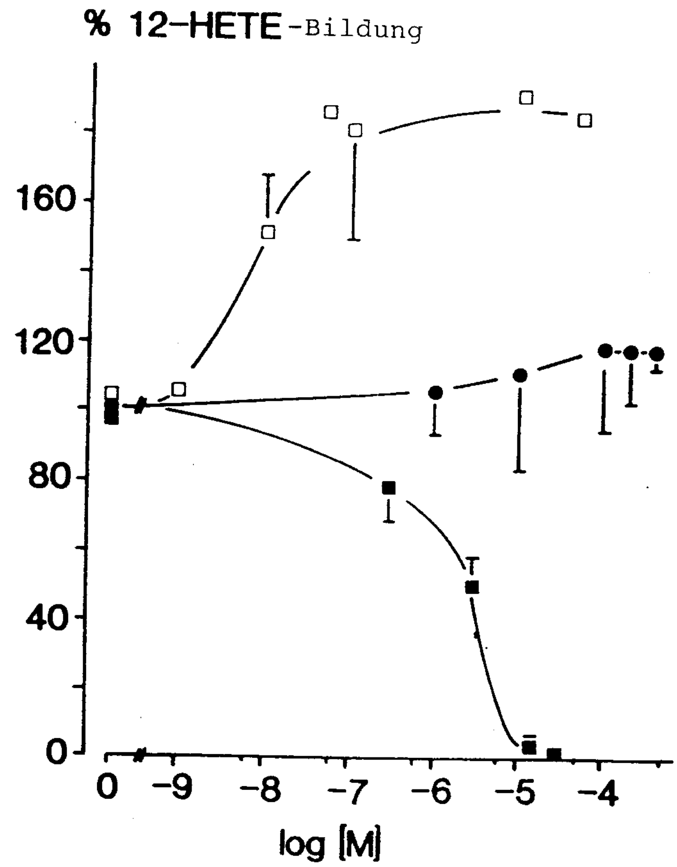
% 12–HETE -Bildung
160
120
80
40
0
0 –9 –8 –7 –6 –5 –4
log [M]

FIG. 3

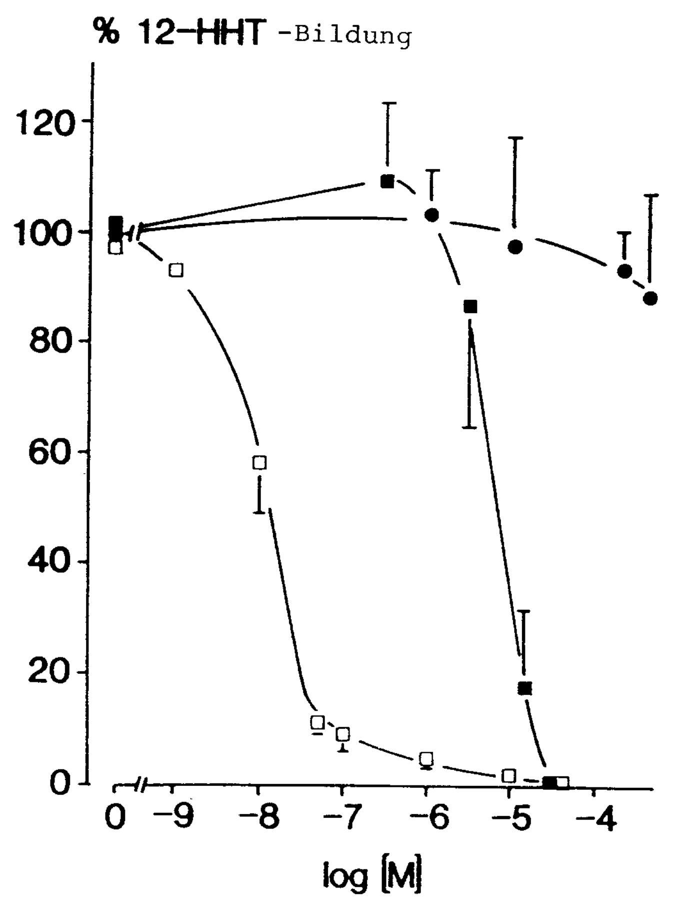
% 12–HHT -Bildung
120
100
80
60
40
20
0
0 –9 –8 –7 –6 –5 –4
log [M]

FIG. 4

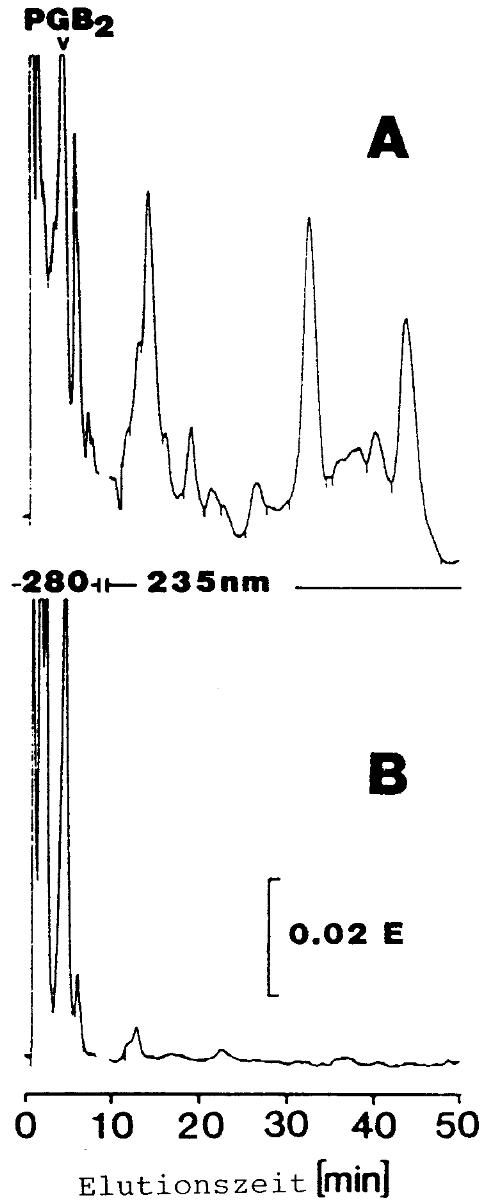

Europäisches Patentamt

# EUROPÄISCHER RECHERCHENBERICHT

Nummer der Anmeldung

EP 93 10 0398
Seite 1

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
| X | INT. J. IMMUNOPHARMACOL.<br>Bd. 11, Nr. 6, 1989,<br>Seiten 647 - 652<br>M.L. SHARMA ET AL 'Antiarthritic activity of boswellic acids in bovine serum albumin(BSA)-induced arthritis.'<br>* Seite 651 - Seite 652 *<br>--- | 1-9 | A61K31/215 |
| X | IND. J .PHARM. SCI.<br>Bd. 52, Nr. 3, 1990,<br>Seiten 158 - 160<br>V. RANGARI ET AL 'Synthesis, antiinflammatory, and antiarthritic activity of newer beta-boswellic acid derivatives'<br>* das ganze Dokument *<br>--- | 1-9 | |
| X | PLANTA MED.<br>Bd. 57, 1991,<br>Seiten 203 - 207<br>H.P.T. AMMON ET AL 'Inhibition of leukotriene B4 formation in rat peritoneal neutrophils by an ethanolic extract of the gum resin exudate of Boswellia serrata'<br>* das ganze Dokument *<br>--- | 1-9 | RECHERCHIERTE SACHGEBIETE (Int. Cl.5 )<br><br>A61K |
| P,X | J. PHARMACOL. EXP. THER.<br>Bd. 261, Nr. 3, 1992,<br>Seiten 1143 - 1146<br>H. SAFAHI ET AL 'Boswellic acids: novel, specific nonredox inhibitors of 5-lipoxygenase'<br>* Seite 1146 *<br>---<br>-/-- | 1-9 | |

Der vorliegende Recherchenbericht wurde für alle Patentansprüche erstellt

| Recherchenort | Abschlußdatum der Recherche | Prüfer |
|---|---|---|
| DEN HAAG | 23 MAERZ 1993 | KLAVER T. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

# EUROPÄISCHER RECHERCHENBERICHT

Nummer der Anmeldung

EP 93 10 0398
Seite 2

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
| A | ITAL. J. BIOCHEM.<br>Bd. 36, Nr. 4, 1987,<br>Seiten 205 - 217<br>G.K. REDDY ET AL 'Effect of a new non-steroidal antiinflammatory agent on lysosomal stability in adjuvant induced arthritis.'<br>--- | | |
| A | WO-A-9 001 937 (RESEARCH TRIANGLE INSTITUTE)<br>----- | | |
| | | | RECHERCHIERTE SACHGEBIETE (Int. Cl.5 ) |

Der vorliegende Recherchenbericht wurde für alle Patentansprüche erstellt

| Recherchenort | Abschlußdatum der Recherche | Prüfer |
|---|---|---|
| DEN HAAG | 23 MAERZ 1993 | KLAVER T. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)